# FASCICULE DE BREVET EUROPEEN

(11) **EP 1 396 533 B1**
(45) Date de publication et mention de la délivrance du brevet: **03.09.2008**
(21) Numéro de dépôt: 02019908.9
(22) Date de dépôt: 04.09.2002
(51) Int. Cl.: C11B 1/10, C11B 1/06, A23D 9/00, C12P 7/64, A23L 1/30, A23K 1/16, A23C 11/04

(54) **Procédé de préparation d'une huile contenant un ou des acides gras polyinsaturé(s) à longue chaine issus de biomasse, aliment, composition nutritionnelle, cosmétique ou pharmaceutique la contenant**
Verfahren zur Herstellung von einem Öl, das langkettige, mehrfach ungesättigte Fettsäuren aus Biomassen enthält, Lebensmittel, Nahrungsmittelzusammensetzung, kosmetische oder pharmazeutische Zusammensetzung, die dieses Ölenthält
Process for preparing an oil containing one or more long chain polyunsaturated fatty acids from biomass, food, nutritional composition, cosmetic or pharmaceutical composition containing said oil

(43) Date de publication de la demande: 10.03.2004
(73) Titulaire: Nestec S.A., 1800 Vevey (CH)
(72) Inventeur: Bertholet, Raymond, 1807 Blonay (CH); Wang, Junkuan, 1027 Lonay (CH); Watzke, Heribert, 1009 Pully (CH); German, Bruce, 1072 Forel/Lavaux (CH)
(74) Mandataire: Dixon, Sarah

(56) Documents cités:
- EP-A- 1 178 118
- EP-A- 1 239 022
- WO-A-92/12086
- WO-A-97/37032
- DATABASE WPI Section Ch, Week 198426 Derwent Publications Ltd., London, GB; Class C03, AN 1984-164291 XP002175534 & SU 1 049 431 A (PLANT PHYSIOL INST), 23 octobre 1983 (1983-10-23)

## Description

### Introduction

La présente invention a trait au domaine de la préparation d'une huile servant d'ingrédient source d'acides gras essentiels polyinsaturés à longue chaîne (LC-PUFA) dans un aliment, dans un supplément nutritionnel, dans une composition cosmétique ou pharmaceutique.

### Etat de la technique

Une huile contenant des LC-PUFA comme par exemple les acides arachidonique (ARA), docosahexaénoique (DHA), eicosapentaenoique (EPA) ou dihomogammalinolénique (DHGLA) peut être obtenue à partir d'un bouillon de fermentation d'une biomasse. Pour obtenir l'huile de la biomasse on a utilisé des méthodes d'extraction par solvant organique, par exemple l'hexane ou par fluide supercritique. Généralement, l'huile a été extraite à partir de biomasse par percolation à l'hexane de la biomasse séchée.

Un tel procédé d'extraction par solvant(s) organique(s) est décrit par exemple dans WO9737032, dans WO 9743362 ou dans la publication Journal of Dispersion Science and Technology, 10, 561-579, 1989 "Biotechnological processes for the production of PUFAs".

Cette technique présente différents inconvénients:
- Lors des étapes d'extraction par solvant chaud et de distillation du solvant les LC-PUFA peuvent subir une dégradation au contact de l'oxygène.
- L'élimination totale du solvant contenu dans l'huile ou dans la biomasse résiduel exige un traitement thermique à température élevée.
- Par ailleurs, le solvant, tel que l'hexane est susceptible de dissoudre des constituants non-triacylglycérols de la biomasse qui constituent en fait des impuretés.

L'huile brute obtenue après évaporation du solvant doit encore subir plusieurs étapes de raffinage comprenant un dégommage, une neutralisation à l'alcali, une décoloration , un décirage et une désodorisation dans le but d'éliminer au moins partiellement les impuretés. Cela implique que l'huile très insaturée est exposée à des conditions stimulant des réactions physico-chimiques qui affectent sa qualité. Par exemple, les agents de décoloration créent un système de doubles liaisons conjuguées et forment des produits de dégradation par réaction chimique avec les glycérides oxydés.

On connait également, par exemple de EP-A-1178118, un procédé d'extraction de biomasse sans solvant. Selon ce procédé, on évite l'utilisation d'un solvant en préparant une suspension aqueuse de la biomasse et en séparant une phase huileuse, contenant l'huile recherchée, de la phase aqueuse, par centrifugation. La phase aqueuse contient des débris de parois cellulaires et quantité de matériau soluble dans l'eau provenant de la biomasse. Un inconvénient de ce procédé est que l'huile brute obtenue est contaminée par des quantités d'impuretés, par exemple des lipides polaires, des résidus de protéines dus à la présence d'eau. Une telle huile brute doit être ensuite raffinée par les méthodes conventionnelles de raffinage des huiles végétales.

La présente invention a pour but d'éviter les inconvénients de l'art antérieur, en proposant un procédé de préparation d'une huile stable contenant un ou des acides gras polyinsaturés issus de biomasse sous forme de triacylglycérols à l'état purifié avec un rendement élevé dans lequel l'huile subit un minimum de dégradation.

### Résumé de l'invention

La présente invention concerne un procédé de préparation d'une huile stable contenant des LC-PUFA sous forme de triacylglycérols, notamment les acides arachidonique (ARA), dihomogammalinolénique (DHGLA), docosahexaénoique (DHA), ou eicosapentaenoique (EPA).

Le procédé est caractérisé par le fait que l'on presse une ou des biomasses provenant de la culture d'un microorganisme, notamment d'un champignon ou d'une microalgue contenant les acides ARA, DHGLA, DHA ou EPA, à sec de manière à obtenir une première huile de pression et un tourteau, que l'on traite l'huile ainsi obtenue avec un adsorbant et qu'on la soumet à une désodorisation dans des conditions ménageantes.

L'huile ainsi obtenue ne nécessite pas de raffinage chimique. Un traitement avec un adsorbant, par exemple un silicate, et une désodorisation dans des conditions douces sont suffisants pour obtenir une huile purifiée et stable pouvant être utilisée en tant que complément nutritionnel.

Selon un mode de mise en oeuvre préféré du procédé,
- on met en contact le tourteau de pressage avec une huile support entrant dans la compostion d'un aliment et on presse le mélange de l'huile support et du tourteau pour obtenir une deuxième huile de pression et
- on mélange les huiles de pression dans des proportions variables de manière à obtenir une concentration en LC-PUFA qui convienne à l'application spécifique. On soumet ensuite, le cas échéant, le mélange à un raffinage physique et à une désodorisation.

De préférence, l'huile ne contient pas plus de 10 % en poids de LC-PUFA. De ce fait, l'huile est beaucoup moins sensible à l'oxydation lors de sa production ce qui n'est pas le cas pour les huiles contenant des LC-PUFA de l'art antérieur.

Selon un aspect principal de l'invention, c'est un avantage qualitatif déterminant de disposer d'une huile nouvelle, contenant des LC-PUFA sous forme de triacylglycérols.

Selon un autre aspect, l'invention concerne un aliment, un produit cosmétique ou pharmaceutique, un supplément nutritionnel ou un aliment pour animaux contenant l'huile précédente.

Selon encore un autre aspect, l'invention concerne un aliment pour animaux, en particulier pour animaux familiers contenant le résidu de biomasse issu du procédé.

### Description détaillée de l'invention

La préparation de l'huile est réalisée par pressage direct d'une biomasse contenant des LC-PUFA et obtention d'une première huile de pression. En vue d'augmenter le rendement en LC-PUFA, on soumet de préférence le tourteau de biomasse issu du pressage direct à un contact avec une huile support et on soumet le mélange à un pressage pour l'obtention d'une deuxième huile de pression. On mélange ensuite les deux huiles de pression dans des proportions variables de manière à obtenir une concentration en LC-PUFA qui convienne à l'application spécifique. On soumet ensuite, le cas échéant, le mélange à un raffinage physique et on obtient ainsi l'huile recherchée.

Par le raffinage physique on vise, dans le contexte de l'invention, une diminution des phospholipides et des acides gras libres et on entend un traitement de dégommage sans utilisation d'acide et sans neutralisation.

A titre d'exemple, on a constaté que dans le cas d'une biomasse contenant l'ARA la première huile de pression était déjà débarrassée de la plupart des acides gras libres et des phospholipides et par suite, selon le degré de pureté recherché, ne nécéssitait pas de traitement de dégommage.

Par contre, dans le cas d'une biomasse contenant du DHA par exemple, un tel traitement de dégommage était nécessaire pour diminuer en particulier les phospholipides en vue d'obtenir le degré de pureté recherché.

L'huile obtenue est adaptée à l'application dans les aliments, en particulier les formules infantiles ou à l'utilisation comme supplément nutritionnel. Elle peut également être utilisée dans des produits cosmétiques ou pharmaceutiques. De plus, le résidu de biomasse obtenu est également un produit du procédé qui peut être valorisé directement sans traitement ultérieur, par exemple comme aliment pour animaux, en particulier pour animaux familiers.

La préparation d'une telle huile peut avoir lieu par simple pressage de la biomasse séchée. De préférence, on procède au mélange de l'huile support avec le résidu du premier pressage de la biomasse et à la séparation ultérieure de l'huile d'avec les solides non lipidiques par pressage.

En vue d'augmenter le rendement en LC-PUFA obtenu, il est préférable de réduire les dimensions des particules de biomasse sèche pour briser les parois des cellules de micoorganismes et libérer ainsi l'huile. On peut dans le mode de réalisation préféré réduire les dimensions des particules du tourteau de pressage pour augmenter la surface de contact entre l'huile support et le résidu de biomasse. Cela peut être réalisé de manière appropriée par utilisation de différentes méthodes, par exemple:
- on peut broyer le tourteau de biomasse en présence de l'huile support ;
- on peut laminer le tourteau de biomasse avant de le mélanger avec l'huile support;
- on peut traiter le tourteau de biomasse sous haute pression en présence de l'huile support ;
   puis séparer l'huile obtenue d'avec le tourteau de biomasse par pressage et filtration de finition.
- on peut traiter la biomasse ou le tourteau avec des enzymes capables de dégrader les parois des cellules.

Du fait que le support est une huile, l'huile obtenue après contact avec le tourteau de biomasse a un contenu minimum en phospholipides, acides gras libres, pigments, polymères et autres substances issues ou dérivées de la biomasse qui ne sont pas des triacylglycérols. Cela signifie que le procédé selon l'invention constitue une méthode sélective de préparation d'une huile purifiée stable contenant des LC-PUFA. Il n'est pas nécessaire de purifier l'huile insaturée contenant les LC-PUFA par les méthodes agressives et lourdes utilisées antérieurement à l'invention que sont les étapes de dégommage, neutralisation, décirage et décoloration.
Selon l'invention, les huiles pressées sont soumises, le cas échéant, à une étape de raffinage à l'aide d'un agent de procédé, par exemple un silicate. Le traitement avec l'agent de procédé peut être réalisé pendant le contact avec l'huile support ou après obtention de l'huile pressée, par exemple lors de la filtration. Finalement les huiles sont soumises à une étape de désodorisation, par exemple par entraînement à la vapeur ou distillation moléculaire à température relativement basse. Il en résulte que l'huile obtenue contient une quantité particulièrement faible d'acides gras trans.

Le procédé n'utilise pas de solvant organique et, dans la mesure où l'on travaille sous couche d'azote et en présence de tocophérols ou tocotrienols naturellement présents ou rajoutés dans l'huile support, les LC-PUFA sont protégés de la dégradation oxydative pendant tout le procédé.

En plus de la qualité de l'huile obtenue, un autre avantage du procédé consiste dans le fait que le résidu de biomasse n'est pas contaminé par un solvant organique et peut ainsi être valorisé directement, sans traitement ultérieur, par exemple dans des aliments pour animaux, notamment pour animaux familiers.

La description détaillée du procédé qui suit est ciblée sur la préparation d'une huile contenant l'ARA, une huile contenant le DHA, et une huile contenant l'ARA et le DHA, pris à titre d'exemples, non limitatifs. Les conditions de travail pour transférer d'autres LC-PUFA vers une huile support à partir des biomasses appropriées, par exemple pour le DHGLA ou EPA sont très proches.

Dans la mise en oeuvre préférée du procédé, l'huile est obtenue par réunion de l'huile de pressage à sec de la biomasse et de l'huile obtenue par mélange de l'huile support avec le résidu de pressage de biomasse sèche et séparation de l'huile d'avec les composants solides par pressage. Pour augmenter le taux d'incorporation en LC-PUFA, il est souhaitable de rompre les cellules microbiennes par des traitements sous haute pression, par des procédés enzymatiques ou de réduire les dimensions des particules sèches de biomasse par mouture ou laminage.

L'étape de mouture mise en oeuvre peut être l'une des nombreuses techniques connues de l'art antérieur. Par exemple, on peut laminer la biomasse, de préférence à basse température, puis on peut la mélanger avec l'huile support. En variante, on peut moudre la biomasse en présence de l'huile support. En vue de minimiser autant que possible la détérioration des LC-PUFA, les conditions de mouture doivent être douces. Dans cette optique, on préfère moudre la biomasse en présence de l'huile support et sous atmosphère inerte, par exemple sous balayage d'azote.

Ensuite, on sépare l'huile contenant les LC-PUFA d'avec le tourteau de la biomasse par filtration ou pressage, de préférence sous forte pression, puis on effectue une filtration de finition de manière à éliminer les particules fines de résidu de biomasse.

On a constaté que le taux d'incorporation en LC-PUFA augmentait lorsque la taille des particules de biomasse diminuait, celui-ci était > 90% lorsque par exemple 90% des particules avaient une dimension < 250 µm.

A titre d'exemple, on peut utiliser un moulin à billes ou un moulin colloïdal. Les paramètres à considérer sont la durée de mouture, la taille des particules de biomasse, la température de mouture, le rapport entre les quantités de biomasse et d'huile support.

La durée de mouture a une influence sur la taille des particules et cette dernière est également influencée par la température de mouture. Par suite, en pratique, on préfère indiquer la taille des particules comme paramètre déterminant de l'étape de mouture. Ainsi, il est souhaitable que 90 % des particules soient de dimension < 500 µm, de préférence que 90 % des particules soient de dimension < 300 µm et de manière encore plus préférée que 90 % des particules soient de dimension < 200 µm.

La température de mouture est choisie à une valeur supérieure au point de fusion de l'huile support, et est de préférence de 20 à 80° C
Le rapport pondéral choisi entre la biomasse et l'huile support détermine le contenu en LC-PUFA de l'huile finale. Ainsi, par exemple, on choisit 30 parties de biomasse pour 70 parties d'huile support pour obtenir au moins 3,5 % de LC-PUFA dans l'huile transformée.

L'huile utilisée comme support peut être n'importe quelle huile ou mélange d'huiles consommable en alimentation humaine. On utilise de préférence une huile ou un mélange entrant dans la composition du produit que l'on souhaite enrichir en PUFA. On peut citer en particulier pour une formule infantile l'huile de tournesol riche en acide oléique (HOSFO), l'huile de tournesol (SFO), l'huile de soja, l'oléine de palme et un triacyglycérol à chaîne moyenne (MCT, contenant essentiellement des triacylglycérols d'acides gras saturés en C₈-C₁₀).

L'étape suivante du procédé consiste à séparer le résidu de biomasse épuisée par toute méthode usuelle comme par exemple le pressage, la filtration ou la centrifugation. A cet effet on utilise de préférence une presse opérant sous forte pression.

L'huile obtenue doit être débarrassée des particules fines insolubles par filtration fine. Cette opération peut être effectuée le cas échéant en mettant l'huile en présence d'un adsorbant minéral en qualité d'aide à la filtration, par exemple la dicalite.

Enfin, l'huile filtrée est désodorisée pour en éliminer les substances volatiles. Cela peut être réalisé par toute méthode connue pourvu que cela soit dans des conditions modérées de manière à ménager les LC-PUFA. On peut citer, par exemple, l'entraînement à la vapeur, de préférence sous vide ou la distillation moléculaire.

L'huile obtenue peut être utilisée dans des compositions alimentaires pour l'alimentation humaine telle quelle ou en mélange avec d'autres huiles, comme par exemple une huile de poisson ou comme par exemple les huilespour salade ou encore sous forme d'émulsion dans les sauces pour salade ou mayonnaises. Elle peut être un constituant d'un lait diététique pour adolescents ou adultes, d'une formule infantile pour prématurés, nourrissons nés à terme ou un lait de suite pour petits enfants.

Elle peut être incorporée dans une composition nutritive ou de supplémentation, pour l'alimenation orale.

Elle peut être incorporée dans une composition pharmaceutique pour l'ingestion orale, entérale ou parentérale, ou pour l'application topique dermatologique ou ophtalmique.

Elle peut constituer un ingrédient d'une composition cosmétique, topique ou orale.

Enfin, elle peut constituer un ingrédient d'un aliment pour animaux familiers, par exemple un aliment sec, humide ou un lait.

Le résidu de biomasse après séparation de l'huile peut avantageusement être utilisé dans les aliments pour animaux, particulièrement les animaux familiers.

### Exemples

Les exemples ci-après illustrent l'invention. Dans ceux-ci, les parties et pourcentages sont pondéraux, sauf indication contraire.
Pour déterminer le % d'acides gras libres (FFA), on utilise la méthode IUPAC 2.201,
pour la teneur en ARA, on utilise la méthode IUPAC 2.304 et
pour la teneur en Phosphore (P), on utilise la méthode NI C12-1976-SSOG.

### Exemple 1: Pressage d'une biomasse contenant ARA avec une presse hydraulique

### Equipement:

Presse Carver RC 106

### Matières utilisées:

Biomasse contenant 34,7 % d'huile à 39,9 % d'acide arachidonique (ARA)

### Procédure :

On introduit 111 g de la biomasse dans une cartouche en carton. La cartouche est introduite dans le cylindre de la presse et le tout est chauffé dans une étuve à 70°C pendant 30 min.
On presse ensuite en augmentant le pression progressivement pour atteindre une pression finale de 700 bar. On recueille ainsi 23,2 g d'huile pressée limpide ce qui correspond à 60,2 % de l'huile présente dans la biomasse.
On détermine la teneur en ARA, acides gras libres (FFA) et Phosphore (P) de l'huile pressée et on la compare avec une huile obtenue après extraction à l'hexane de la même biomasse. Les résultats sont résumés dans le tableau 1 ci-après.

**Tableau 1**

| | Huile de pressage direct | Huile extraite à l'hexane |
|---|---|---|
| % ARA | 39,9 | 39,9 |
| % FFA | 0,1 | 0,6 |
| mg/kg P | 17 | 508 |

L'huile obtenue après pressage direct a une teneur en ARA similaire à l'huile extraite avec l'hexane mais contient moins d'impuretés (FFA, P). L'huile pressée ne nécessite pas de raffinage chimique au contraire de l'huile extraite classiquement avec l'hexane. Un traitement avec un adsorbant (agent de procédé) suivi d' une désodorisation permettent d'obtenir une huile stable et d'odeur neutre.

### Exemple 2: Pressage d'une biomasse contenant DHA avec une presse à vis

### Equipement:

Presse à vis Komet
Filtre-presse Seitz

### Matières utilisées:

Biomasse contenant 50,5 % d'huile à 39 % d'acide docosahexaénoïque (DHA)

### Procédure :

150 kg de biomasse ont été pressés sur la presse Komet dans les conditions suivantes:
fente: 8 mm, débit: 3,75 kg/h

On recueille une huile trouble qui est filtrée à l'aide d'un filtre papier 20 microns. On obtient ainsi 46 kg d'une huile limpide ce qui correspond à 60,7 % de l'huile présente dans la biomasse.
On détermine la teneur en DHA, acides gras libres (FFA) et Phosphore (P) de l'huile pressée et on la compare avec une huile obtenue après extraction à l'hexane de la même biomasse. Les résultats sont résumés dans le tableau 2 ci-après.

**Tableau 2**

| | Huile pressage direct | Huile extraite à l'hexane |
|---|---|---|
| % DHA | 39 | 39 |
| % FFA | 0,7 | 1,6 |
| mg/kg P | 200 | 630 |

L'huile obtenue après pressage direct à une teneur en DHA similaire à l'huile extraite avec l'hexane mais contient moins d'impuretés (FFA, P). Les pertes lors du raffinage seront moindres. Un traitement avec un adsorbant (agent de procédé) suivi d'une désodorisation permettent d'obtenir une huile stable et d'odeur neutre.

### Exemple 3: Incorporation du DHA résiduel de la biomasse pressée dans de l'oléine de palme

### Equipement:

Broyeur colloïdal Fryma MZ 80
Unité de filtration De Smet minipilote avec chambre 1,4 l
Filtre-presse Seitz 2 l
Désodoriseur de laboratoire

### Matières utilisées:

Biomasse DHA pressée de l'exemple 2 contenant 28,2 % d'huile à 39 % d'acide decosahexaénoïque (DHA)
Oléine de palme
Trisyl® (agent de procédé)

### Procédure :

On introduit 1,4 kg de la biomasse pressée telle obtenue dans l'exemple 2 et 2 kg d'oléine de palme dans le récipient du broyeur. Le broyage s'effectue par un passage direct à travers le broyeur avec une fente de 10 microns à une tempétature de 40-50°C et on récupère le mélange. On introduit 2,6 kg de ce mélange dans l'unité de filtration et on procède à une filtration à 50°C, puis le tourteau est lavé par injection de 0,5 kg d' oléine de palme. Le tourteau est ensuite pressé jusqu'à un pression de 15 bar. On recueille ainsi 2 kg d'une huile légèrement trouble qui est filtrée sur le filtre-presse avec un filtre de 1 micron. On détermine le teneur en P de l'huile filtrée qui s'élève à 66 mg/kg.
L'huile filtrée est soumise à un raffinage physique. Dans le but de diminuer la teneur en phosphore, l'huile est agitée à 85 °C pendant 20 min. avec 2 % de Trisyl®, puis séchée à 85°C sous vide 20 mbar et filtrée. Finalement, l'huile est désodorisée à 180°C / 1 mbar pendant 3 heures. L'huile raffinée est analysée afin de déterminer sa pureté (FFA, P) et le taux d'incorporation du DHA. Les résultats sont résumés dans le tableau 3 ci-après.

**Tableau 3**

| | |
|---|---|
| % DHA | 4,9 |
| Taux d'incorporation du DHA (%) | 96,5 |
| % FFA | 0,07 |
| mg/kg P | 2 |

### Exemple 4: Raffinage physique du mélange d'huiles pressées contenant le DHA

### Equipement:

Réacteur en verre de laboratoire 1 l
Filtre-presse Seitz 2 l
Désodoriseur de laboratoire

### Matières utilisées:

Trisyl® (agent de procédé)

### Procédure :

Dans le but d'obtenir une huile raffinée avec une concentration en DHA d'environ 8 %, on mélange dans le réacteur 50 g d'huile pressée telle obtenue dans l'exemple 2 et 500 g d'huile pressée telle obtenue dans l'exemple 3 avant l'étape de raffinage. Le mélange est agité à 85 °C pendant 20 min. avec 2 % de Trisyl®, puis séché à 85 °C sous vide 20 mbar, et finalement filtré. On recueille ainsi 540 g d'huile limpide.
On intoduit 300 g de cette huile dans le désodoriseur et on traite à la vapeur pendant 3 heures à 180 °C / 1 mbar. L'huile raffinée est analysée afin de déterminer sa pureté (FFA, P) et la teneur en DHA. Les résultats sont résumés dans le tableau 4 ci-après.

**Tableau 4**

| | |
|---|---|
| % DHA | 8 |
| % FFA | 0,07 |
| mg/kg P | 2 |

### Exemple 5: Incorporation dans de l'oléine de palme de DHA et de ARA provenant de biomasses

### Equipement:

Broyeur colloïdal Fryma MZ 80
Unité de filtration De Smet minipilote avec chambre 1,4 l
Filtre-presse Seitz 2 l
Désodoriseur de laboratoire

### Matières utilisées:

Biomasse contenant 36 % d'huile à 43,1 % d'acide arachidonique (ARA)
Biomasse contenant 50,5 % d'huile à 39% d'acide decosahexaénoïque (DHA)
Oléine de palme
Trisyl® (agent de procédé)

### Procédure:

On introduit 0,7 kg de la biomasse contanant le DHA, 0,7 kg de la biomasse contenant l'ARA et 2 kg d'oléine de palme dans le récipient du broyeur. Le broyage s'effectue par un passage direct à travers le broyeur avec une fente de 10 microns à une tempétature de 40-50°C et on récupère le mélange. On introduit 2,5 kg de ce mélange dans l'unité de filtration et on procède à une filtration à 50°C. Le tourteau est lavé par injection de 0,92 kg d' oléine de palme. Le tourteau est ensuite pressé jusquà un pression de 15 bar. On recueille ainsi 2,5 kg d'une huile légèrement trouble qui est filtrée sur le filtre-presse avec un filtre de 1 micron. On détermine le teneur en P de l'huile filtrée qui s'élève à 26 mg/kg.
L'huile filtrée est soumise à un raffinage physique. Dans le but de diminuer la teneur en phosphore, l'huile est agitée à 85 °C pendant 20 min. avec 1 % de Trisyl®, séchée à 85°C sous vide 20 mbar, puis filtrée. Finalement, l'huile est désodorisée à 180°C / 1 mbar pendant 3 heures. L'huile raffinée est analysée afin de déterminer sa pureté (FFA, P) et le taux d'incorporation du DHA et de l'ARA. Les résultats sont résumés dans le tableau 5 ci-après.

**Tableau 5**

| | |
|---|---|
| % DHA | 2,3 |
| Taux d'incorporation du DHA (%) | 64,6 |
| % ARA | 2,8 |
| Taux d'incorporation de l'ARA (%) | 99 |
| % FFA | 0,04 |
| mg/kg P | 2 |

### Exemples 6-8

6. On prépare une formule infantile pour prématurés enrichie en ARA à partir de huile préparée par le procédé de l'exemple 1 et une huile de poisson enrichie en DHA, contenant env. 24 % de DHA et on y ajoute d'autres huiles par exemple dans les proportions indiquées dans le tableau 6 ci-dessous, des protéines, le cas échéant hydrolysées, des hydrates de carbone et le cas échéant des vitamines et des oligoéléments.

**Tableau 6**

| | Exemple 6 |
|---|---|
| Huile de l'exemple 1 | 0,8 |
| Huile de poisson | 1,3 |
| Huile MCT | 27 |
| Huile de soja | 23 |
| Oléine de palme | 47,9 |
| Total | 100 |

7. On prépare une formule infantile pour nourrissons nés à terme enrichie en ARA et en DHA à partir de l'huile support préparée par le procédé de l'exemple 5 et on y ajoute d'autres huiles par exemple dans les proportions indiquées dans le tableau 7 ci-dessous, des protéines, le cas échéant hydrolysées, des hydrates de carbone et le cas échéant des vitamines et des oligoéléments.

**Tableau 7**

| | Exemple 7 |
|---|---|
| Huile de l'exemple 5 | 13 |
| Huile de coco | 20 |
| Huile de soja | 20 |
| Oléine de palme | 47 |
| Total | 100 |

8. On prépare un lait de suite pour petits enfants enrichi en DHA à partir de l'huile support préparée par le procédé de l'exemple 4, et on y ajoute d'autres huiles dans les proportions indiquées dans le tableau 8 ci-dessous, des protéines, le cas échéant hydrolysées, des hydrates de carbone et le cas échéant des vitamines et des oligoéléments.

**Tableau 8**

| | Exemple 8 |
|---|---|
| Huile de l'exemple 4 | 4 |
| Huile de palmiste | 27 |
| Huile de soja | 23 |
| Oléine de palme | 46 |
| Total | 100 |

### Exemple 9

On prépare un lait liquide enrichi en DHA à raison de 1 % de DHA dans la phase grasse de la manière suivante:
On pasteurise séparément un lait entier contenant 3,92 % de matière grasse et 8,58 % de solides non gras et un lait maigre contenant 0,05 % de matière grasse et 9 % de solides non gras, en les traitant à 87° C pendant 12 s.

On mélange ensuite 34,69 kg de lait entier et 160,26 kg de lait maigre, refroidi à 15° C, puis on incorpore à ce mélange un prémélange de 0,77 kg d'huile obtenue selon l'exemple 3 (oléine de palme, contenant 4,9 % de DHA), 1,6 kg d'huile de soja et 1 g de vitamine E porté à 50° C au moyen d'un moulin colloïdal.

### Produit stérilisé:

Après réchauffage à 80° C dans un échangeur à plaques, on stérilise le liquide par UHT à 148° C pendant 5 s. Après refroidissement à 78° C, on l'homogénéise en deux étages, à 200 bar, puis à 50 bar, on le refroidit à 20° C et on le conditionne aseptiquement dans des emballages de type brique ayant été préalablement stérilisés, les étapes d'homogénéisation, de refroidissement et de remplissage ayant lieu de manière aseptique.

### Produit pasteurisé:

On chauffe le liquide à 72° C pendant 15 dans un échangeur à plaques, on l'homogénéise en deux étages à 200 bar, puis à 50 bar, on le refroidit à 4° C et on le conditionne dans des emballage de type brique.

### Exemple 10

A titre de supplément nutritionnel, on encapsule une huile préparée selon l'exemple 1 contenant l'ARA ou une huile préparée selon l'exemple 2, contenant le DHA à raison de 500 mg d'huile dans des gélules.

## Revendications

1. Procédé de préparation d'une huile stable contenant des acides gras polyinsaturés à longe chaîne (LC-PUFA) sous forme de triacylglycérols, notamment les acides arachidonique (ARA), dihomogammalinolénique (DHGLA), docosahexaénoique (DHA), ou eicosapentaenoique (EPA), **caractérisé par le fait que** l' on presse une ou des biomasses provenant de la culture d'un microorganisme, notamment d'un champignon ou d'une microalgue contenant les acides ARA, DHGLA, DHA ou EPA, à sec de manière à obtenir une première huile de pression et un tourteau, que l'on traite l'huile ainsi obtenue avec un adsorbant et qu'on la soumet à une désodorisation dans des conditions ménageantes.

2. Procédé selon la revendication 1, dans lequel la biomasse contient au moins une acide gras polyinsaturé à longue chaîne choisi parmi l'ARA et le DHA.

3. Procédé selon la revendication 2, dans lequel on traite une biomasse contenant l'ARA.

4. Procédé selon la revendication 2, dans lequel on traite une biomasse contenant le DHA.

5. Procédé selon la revendication 2, dans lequel on traite un mélange de biomasses, contenant l'ARA et le DHA.

6. Procédé selon l'une des revendications 1 à 5, dans lequel on met en contact une huile support, entrant dans la composition d'un produit alimentaire, nutritionnel, pharmaceutique ou cosmétique, avec le tourteau de pression de la biomasse de manière à transférer le ou les acides gras polyinsaturés à longue chaîne sous forme de triacylglycérols vers le dit support, on sépare l'huile contenant le ou les dits acides gras du tourteau de biomasse par pressage et filtration, qui constitue la deuxième huile de pression, on réunit les huiles pressées et on les raffine dans des conditions ménageantes.

7. Procédé selon la revendication 6, dans lequel on soumet les huiles pressées à un raffinage physique à l'aide d'un agent de procédé, le traitement pouvant être réalisé pendant le contact avec l'huile support ou après obtention de huile pressée, notamment lors de la filtration.

8. Procédé selon la revendication 1, dans lequel on brise les parois des cellules des microorganismes par pressage, pour augmenter le taux d'incorporation de l'huile de biomasse dans l'huile support. ,

9. Procédé selon la revendication 6, dans lequel on soumet le tourteau de pression de la biomasse à une mouture en présence de l'huile support dans des conditions douces, à température modérée sous atmosphère inerte, notamment sous couche d'azote.

10. Procédé selon l'une des revendications 6 à 9, dans lequel on effectue une filtration de finition de manière à éliminer les particules fines de résidu de biomasse.

11. Aliment contenant une huile obtenue par le procédé selon l'une des revendications 1 à 10.

12. Aliment infantile contenant une huile obtenue par le procédé selon l'une des revendications 1 à 10.

13. Aliment infantile contenant une huile obtenue par le procédé selon l'une des revendications 1 à 10, en combinaison avec une huile de poisson.

14. Composition nutritionnelle contenant une huile obtenue par le procédé selon l'une des revendications 1 à 10.

15. Composition cosmétique sous forme sèche ou d'émulsion contenant une huile obtenue par le procédé selon l'une des revendications 1 à 10.

16. Aliment contenant une huile obtenue selon l'une des revendications 1 à 10 destiné à l'alimentation des animaux .

17. Aliment contenant le résidu de la biomasse obtenu par le procédé selon l'une des revendications 1 à 10, destiné à l'alimentation des animaux.

## Claims

1. Process for preparing a stable oil containing long-chain polyunsaturated fatty acids (LC-PUFA) in the form of triacylglycerols, in particular arachidonic (ARA), dihomogammalinolenic (DHGLA), docosahexaenoic (DHA) or eicosapentaenoic (EPA) acids, **characterised in that** one or more biomasses originating from the culture of a micro-organism, in particular a fungus or a micro-alga containing the acids ARA, DHGLA, DHA or EPA, is dry-pressed so as to obtain a first press oil and a cake, **in that** the oil thus obtained is treated with an adsorbent, and **in that** said oil is subjected to deodorisation under controlled conditions.

2. Process according to claim 1, wherein the biomass contains at least one long-chain polyunsaturated fatty acid selected from ARA and DHA.

3. Process according to claim 2, wherein a biomass containing ARA is treated.

4. Process according to claim 2, wherein a biomass containing DHA is treated.

5. Process according to claim 2, wherein a mixture of biomasses, containing ARA and DHA, is treated.

6. Process according to any one of claims 1 to 5, wherein a carrier oil, found in the composition of a food, nutritional, pharmaceutical or cosmetic product, is brought into contact with the press cake of the biomass in such a way as to transfer the long-chain polyunsaturated fatty acid(s) in the form of triacylglycerols towards said carrier, the oil containing said fatty acid(s) is separated from the biomass cake by pressing and filtration, forming the second press oil, and the pressed oils are brought together again and refined under controlled conditions.

7. Process according to claim 6, wherein the pressed oils are subjected to physical refinement using a processing agent, it being possible to carry out the treatment during the contact with the carrier oil or after obtaining the pressed oil, in particular during filtration.

8. Process according to claim 1, wherein the cell walls of the micro-organisms are broken by pressing in order to increase the extent to which the biomass oil is incorporated into the carrier oil.

9. Process according to claim 6, wherein the press cake of the biomass is subjected to grinding in the presence of the carrier oil under gentle conditions, at a moderate temperature under an inert atmosphere, in particular under a nitrogen layer.

10. Process according to any one of claims 6 to 9, wherein a final filtration is carried out in such a way as to remove the fine particles of biomass residue.

11. Foodstuff containing an oil obtained by the process according to any one of claims 1 to 10.

12. Infant foodstuff containing an oil obtained by the process according to any one of claims 1 to 10.

13. Infant foodstuff containing an oil obtained by the process according to any one of claims 1 to 10, combined with a fish oil.

14. Nutritional composition containing an oil obtained by the process according to any one of claims 1 to 10.

15. Cosmetic composition in dry or emulsion form containing an oil obtained by the process according to any one of claims 1 to 10.

16. Foodstuff intended for feeding animals and containing an oil obtained by the process according to any one of claims 1 to 10.

17. Foodstuff intended for feeding animals and containing the residue, obtained by the process according to any one of claims 1 to 10, of the biomass.

## Patentansprüche

1. Verfahren zur Herstellung eines stabilen Öls, das langkettige, mehrfach ungesättigte Fettsäuren (LCPUFA) in Form von Triacylglycerinen enthält, insbesondere Arachidonsäure (ARA), Dihomogammalinolensäure (DHGLA), Docosahexaensäure (DHA) oder Eicosapentaensäure (EPA), **dadurch gekennzeichnet, dass** man eine oder mehrere Biomassen, die von der Kultur eines Mikroorganismus, insbesondere eines Pilzes oder einer Mikroalge, stammen, der die Säuren ARA, DHGLA, DHA oder EPA enthält, trocken so presst, dass man ein erstes Pressöl und einen Presskuchen erhält, dass man das auf diese Weise erhaltene Öl mit einem Adsorptionsmittel behandelt und dass man es einer Deodorierung bei schonenden Bedingungen unterzieht.

2. Verfahren nach Anspruch 1, bei dem die Biomasse mindestens eine langkettige, mehrfach ungesättigte Fettsäure enthält, die aus ARA und DHA ausgewählt ist.

3. Verfahren nach Anspruch 2, bei dem man eine ARA enthaltende Biomasse behandelt.

4. Verfahren nach Anspruch 2, bei dem man eine DHA enthaltende Biomasse behandelt.

5. Verfahren nach Anspruch 2, bei dem man eine Mischung von Biomassen behandelt, die ARA und DHA enthält.

6. Verfahren nach einem der Ansprüche 1 bis 5, bei dem man ein Trägeröl, das in der Zusammensetzung eines Nahrungsmittelprodukts, eines Nährmittelprodukts, eines pharmazeutischen oder kosmetischen Produkts enthalten ist, mit dem Presskuchen der Biomasse so in Kontakt bringt, dass die langkettige, mehrfach ungesättigte Fettsäure bzw. die langkettigen, mehrfach ungesättigten Fettsäuren in Form von Triacylglycerinen auf den Träger übertragen werden, man das die Fettsäure oder die Fettsäuren enthaltende Öl vom Biommassekuchen durch Pressen und Filtrieren trennt, welches das zweite Pressöl bildet, man die gepressten Öle vereinigt und sie bei schonenden Bedingungen raffiniert.

7. Verfahren nach Anspruch 6, bei dem man die gepressten Öle einer physikalischen Raffinierung mit Hilfe eines Verfahrensmittels unterzieht, wobei die Behandlung während des Kontakts mit dem Trägeröl oder nach Erhalten des gepressten Öls, insbesondere bei der Filtration, durchgeführt werden kann.

8. Verfahren nach Anspruch 1, bei dem man die Wände der Zellen der Mikroorganismen durch Pressen bricht, um den Grad der Einarbeitung des Biomasseöls in das Trägeröl zu erhöhen.

9. Verfahren nach Anspruch 6, bei dem man den Presskuchen der Biomasse einer Mahlung in Anwesenheit des Trägeröls bei milden Bedingungen bei einer mäßigen Temperatur unter Inertatmosphäre, insbesondere unter einer Stickstoffschicht, unterzieht.

10. Verfahren nach einem der Ansprüche 6 bis 9, bei dem man eine Endfiltration so durchführt, dass die feinen Biomasserückstandteilchen entfernt werden.

11. Nahrungsmittel, das ein in dem Verfahren nach einem der Ansprüche 1 bis 10 erhaltenes Öl enthält.

12. Babynahrung, die ein in dem Verfahren nach einem der Ansprüche 1 bis 10 erhaltenes Öl enthält.

13. Babynahrung, die ein in dem Verfahren nach einem der Ansprüche 1 bis 10 erhaltenes Öl in Kombination mit einem Fischöl enthält.

14. Nährzusammensetzung, die ein in dem Verfahren nach einem der Ansprüche 1 bis 10 erhaltenes Öl enthält.

15. Kosmetische Zusammensetzung in trockener Form oder in Form von Emulsion, die ein in dem Verfahren nach einem der Ansprüche 1 bis 10 erhaltenes Öl enthält.

16. Nahrungsmittel, das ein gemäß einem der Ansprüche 1 bis 10 erhaltenes Öl enthält, für die Ernährung von Tieren.

17. Nahrungsmittel, das den in dem Verfahren nach einem der Ansprüche 1 bis 10 erhaltenen Rückstand der Biomasse enthält, für die Ernährung von Tieren.
